# Europäisches Patentamt
# European Patent Office
# Office européen des brevets

(11) Veröffentlichungsnummer : **0 027 291**
**B1**

(12) **EUROPÄISCHE PATENTSCHRIFT**

(45) Veröffentlichungstag der Patentschrift :
**14.09.83**

(21) Anmeldenummer : **80200947.2**

(22) Anmeldetag : **08.10.80**

(51) Int. Cl.³ : **A 61 B   6/02**, A 61 B   6/06

(54) Vorrichtung zur Erzeugung von Schichtbildern aus Vielfachperspektivbildern mit unterschiedlichem Überlagerungsgrad.

(30) Priorität : **12.10.79 DE 2941395**

(43) Veröffentlichungstag der Anmeldung :
**22.04.81 Patentblatt 81/16**

(45) Bekanntmachung des Hinweises auf die Patenterteilung : **14.09.83 Patentblatt 83/37**

(84) Benannte Vertragsstaaten :
**BE DE FR GB NL SE**

(56) Entgegenhaltungen :
DE B 2 514 988
FR A 1 265 256
FR A 1 336 114
US A 4 097 748
US A 4 138 721

(73) Patentinhaber : **Philips Patentverwaltung GmbH**
**Steindamm 94**
**D-2000 Hamburg 1 (DE)**
**DE**
**N.V. Philips' Gloeilampenfabrieken**
**Groenewoudseweg 1**
**NL-5621 BA Eindhoven (NL)**
**BE FR GB NL SE**

(72) Erfinder : **Weiss, Hermann, Dr.**
**Mesterbrooksweg 2**
**D-2000 Hamburg 65 (DE)**
Erfinder : **Linde, Rolf**
**Kamperrege 21**
**D-2081 Haseldorf (DE)**
Erfinder : **Mauser, Wilfried**
**Wohlers Allee 7**
**D-2000 Hamburg 50 (DE)**
Erfinder : **Klotz, Erhard**
**Seekamp 110**
**D-2083 Halstenbek (DE)**

(74) Vertreter : **Hartmann, Heinrich, Dipl.-Ing. et al**
**Philips Patentverwaltung GmbH Steindamm 94**
**D-2000 Hamburg 1 (DE)**

Jouve, 18, rue St-Denis, 75001 Paris, France

Vorrichtung zur Erzeugung von Schichtbildern aus Vielfachperspektivbildern mit unterschiedlichem Überlagerungsgrad

Die Erfindung betrifft eine Vorrichtung zur Erzeugung von Schichtbildern eines dreidimensionalen Objektes, mit in einer Strahlenquellenebene angeordneten Strahlenquellen zur Durchstrahlung des Objektes mit aus unterschiedlichen Richtungen verlaufenden Strahlenbündeln, und mit mehreren, unterhalb des Objektes und parallel zueinander angeordneten, ebenen Aufzeichnungsschichten zur Aufnahme jeweils aller Perspektivbilder.

Eine derartige Vorrichtung ist aus der DE-A-25 14 998 bereits bekannt. Bei dieser Vorrichtung werden mit einer Röntgenröhren enthaltenden Mehrfachstrahlenquelle kodierte Bilder auf mehreren hintereinander liegenden Filmen aufgenommen, wobei die kodierten Bilder jeweils aus sich mehr oder weniger überlagernden Perspektivbildern bestehen. Aus diesen kodierten Bildern können in einem weiteren Schritt Schichtbilder des Objektes rekonstruiert werden (siehe DE-B-27 19 386 und 27 46 035).

Aus der US-A-4 097 748 ist eine Vorrichtung zum Erzeugen von Schichtbildern mit nur einer ebenen Aufzeichnungsschicht bekannt, vor der eine ihr zugeordnete Blendenanordnung angeordnet ist.

Da die Schichtbilder (DE-A-25 14 998) im Prinzip aus den überlagerten Perspektivbildern entstehen, diese jedoch bei den kodierten Bildern nicht in getrennter, sondern in überlagerter Form vorliegen, werden bei der Schichtdarstellung, bedingt durch diese Überlagerungen, zusätzliche Artefakte ins Schichtbild übertragen, die die Bildqualität verschlechtern. Mit zunehmendem Überlagerungsgrad wird dabei der Einfluß der Artefakte größer. Die Artefakte können zwar verwischt werden ; das Verfahren erfordert jedoch Mehrkanal-Dekodiervorrichtungen zur Dekodierung der in den jeweiligen Ebenen aufgezeichneten, überlagerten Perspektivbilder.

Durch getrennte Perspektivbilder in jeweils einer Aufzeichnungsebene können die zuvor beschriebenen Artefakte jedoch vermieden und so die Bildqualität wesentlich verbessert werden. Die Objektgröße ist aber bei getrennten Bildern stark eingeschränkt, wenn nicht allzu große Filmformate verwendet werden sollen. Bei einer technisch realisierbaren Strahlengeometrie (ca. 25 Röhren, Fokus-Objekt-Abstand FOA = 1 200 mm (Objekt-Film-Abstand OFA = 500 mm)) und einem handelsüblichen Filmformat 40 × 40 cm² können daher lediglich Objekte mit einem Durchmesser von ca. 50 mm in getrennter Form aufgezeichnet werden. Bei Objekten von dieser Größe ist es aber für den Arzt sehr schwierig, eine genaue Diagnose zu stellen, weil bei dieser Größe eine Orientierung nach benachbarten, bekannten und größeren Objektdetails (z. B. mit Kontrastmittel gefüllte Gefäße), die für den Arzt eine wichtige Hilfe bei seiner Diagnosefindung sein können, meistens nicht möglich ist, da diese Information in den Bildern fehlt oder aber an ungünstiger Stelle, z. B. am Bildrand, liegt. Es ist daher wünschenswert, zuerst einmal ein Schichtbild von einem relativ großen Objektbereich herzustellen, anhand dessen sich der Arzt orientieren kann. Durch eine zusätzliche Aufnahme wird aber für den Patienten die Strahlenbelastung größer. Gleiches gilt auch für die Belastung durch Kontrastmittel, wenn diese zur Aufnahme gespritzt werden müssen. Nachteilig für Patient und Arzt ist natürlich auch die sich daraus ergebende längere Untersuchungsdauer.

Aufgabe der Erfindung ist es daher, eine Vorrichtung zur Erzeugung von Schichtbildern zu schaffen, mit deren Hilfe sowohl kodierte Bilder großer Objektbereiche bei relativ starker Überlagerung der Perspektivbilder als auch gleichzeitig kodierte Bilder mit getrennten Perspektivbildern hergestellt werden können, die nur einen Teil des Objektbereiches darstellen.

Diese Aufgabe wird gemäß der Erfindung dadurch gelöst, daß vor jeweils einer Aufzeichnungsschicht eine ihr zugeordnete Blendenanordnung zur stufenweisen Ausblendung der Strahlenbündel angeordnet ist, wobei die Blendenöffnungen jeweils einer Blendenanordnung umso kleiner sind, je weiter die Blendenanordnung vom Objekt entfernt ist.

Bei dieser Vorrichtung werden die mit der Mehrfachstrahlenquelle erzeugten Perspektivbilder in einem untereinander geschichteten Satz von Filmen (Aufzeichnungsschichten) aufgezeichnet, bei dem Film um Film der Objektbereich und die damit verbundene Überlagerung der Perspektivbilder kleiner wird.

Hierdurch wird erreicht, daß von einem Objekt bei Bestrahlung eines relativ großen Objektbereiches auf einem ersten Film Perspektivbilder aufgezeichnet werden können, die sich zwar stark überlagern, an denen aber eine erste Orientierung bzw. eine Vordiagnose des Patienten vorgenommen werden kann. Gleichzeitig wird, ohne daß der Patient bzw. das Objekt nochmals bestrahlt werden muß, von einem kleineren Bereich des Objektes ein weiteres kodiertes Bild mit Perspektivbildern erzeugt, die sich jetzt nicht mehr oder nur wenig überlagern. Aus diesen kodierten Bildern können dann jeweils artefaktarme Schichtbilder rekonstruiert werden. Wichtig ist hierbei, daß das medizinisch relevante Objektdetail noch in dem abgeblendeten Objektbereich liegt.

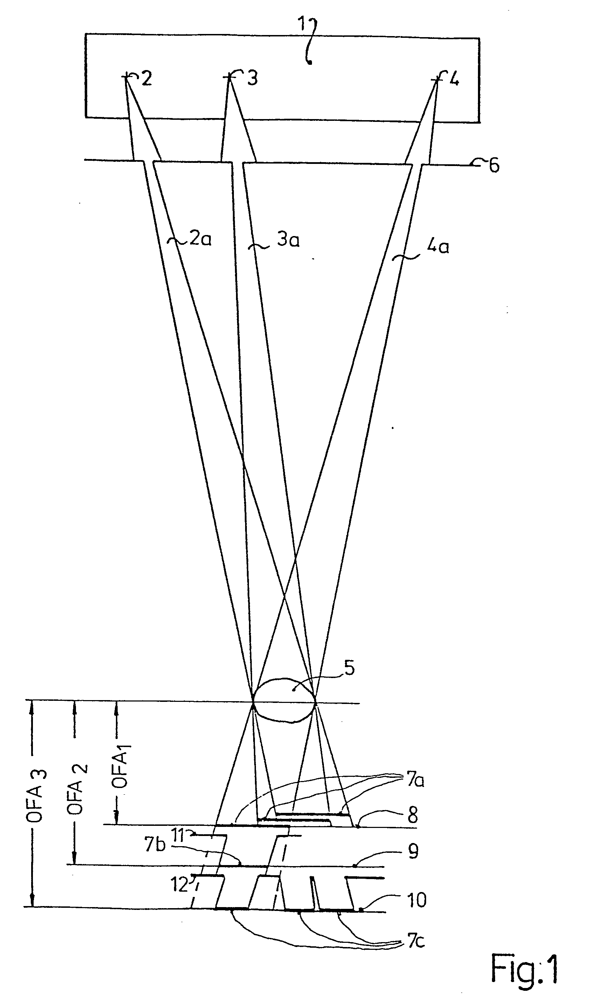

Die Figur 1 stellt ein Ausführungsbeispiel der Erfindung dar.

Sie zeigt eine Mehrfachstrahlenquelle 1 mit drei Röntgenröhren 2, 3 und 4. Das Objekt 5 wird mit einer Blendenanordnung 6, z. B. einer Blendenplatte, ausgeblendet. Dabei können die Strahlenbündel 2a, 3a, 4a derart begrenzt sein, daß ein möglichst großer Objektbereich durchstrahlt wird, bzw. die verwendeten Filmformate vollständig belichtet werden. Die Blende 6 kann dabei mit verschiedenen oder gleich großen Blendenöffnungen versehen sein. Verschieden große Blendenöffnungen können z. B. verwendet werden, wenn die Perspektivbilder in wenig überlagerter Form und möglichst gleichmäßig über einen Film verteilt aufgenommen werden sollen. Ferner ist natürlich auch unterhalb des Objektes, also zwischen Objekt und erster Aufzeichnungsschicht, eine weitere Blende zur Ausblendung der Primärstrahlenbündel angeordnet (nicht dargestellt).

Die Perspektivbilder 7a werden auf einem Film 8 im Abstand OFA$_1$ aufgezeichnet. Bei diesem Abstand und bei diesem ausgeblendeten Objektbereich werden die einzelnen Perspektivbilder stark überlagert. Die Folge davon ist eine reduzierte Bildqualität des rekonstruierten Schichtbildes. Im Abstand OFA$_2$ und OFA$_3$ z. B. sind gleichzeitig weitere Filme 9 und 10 angeordnet, auf denen mit Hilfe von dazwischen angeordneten Blenden (z. B. Bleiblenden) 11 und 12 die stärker ausgeblendeten Perspektivbilder 7b und 7c aufgezeichnet werden. Aus Gründen der Übersichtlichkeit wurden nur für die Strahlenquelle 4 die vollständigen Strahlengänge gezeichnet. Die Blendenöffnungen der Blendenanordnungen 11 bzw. 12 werden dabei mit zunehmendem Objekt-Film-Abstand kleiner. In diesem Beispiel sind daher im Abstand OFA$_3$ die Perspektivbilder 7c getrennt aufgezeichnet. Die Ausblendung kann z. B. zentrisch zu den Primärstrahlenbündeln 2a, 3a, 4a, aber auch in jeder anderen beliebigen Form durchgeführt werden. Außerdem kann die Ausblendung für die einzelnen Perspektivbilder auch unterschiedlich sein, das Ziel ist immer, eine geringe oder keine Überlagerung der Perspektivbilder zu erreichen. Ohne die Blendenanordnung 11 bzw. 12 würde für die Röntgenquelle 4 der gestrichelt eingezeichnete Strahlenverlauf gelten.

In diesem Beispiel ist der Objekt-Film-Abstand OFA$_1$ so gewählt, daß das Filmformat des Filmes 8 optimal, d. h. bis zum Rand, ausgenutzt wird. Die Filme 8, 9 und 10 sind nach bekannter Art beidseitig mit Verstärkerfolien kombiniert; der Übersichtlichkeit halber wurden diese jedoch nicht dargestellt. Durch geeignete Anpassung der Verstärkerfolien (Verstärkungsfaktor) kann man erreichen, daß die variierende Bildinformation (Überlagerungsgrad, Objekt-Film-Abstand) auf den verschiedenen Filmen 8, 9 und 10 annähernd mit gleicher Schwärzung aufgezeichnet wird. Der Verstärkungsfaktor ist vom Film 8 nach 10 steigend. Die Anpassung kann prinzipiell auch mit Filmen unterschiedlicher Empfindlichkeit durchgeführt werden. Bei Verwendung dieses Filmsatzes erhöht sich die Strahlendosis für den Patienten nur unwesentlich.

Die Filme 7, 8 und 9 befinden sich vorzugsweise in einer gemeinsamen Filmkassette (Simultan-Kassette) oder werden mit Hilfe einer Filmwechselvorrichtung gleichzeitig in ihre jeweilige Position gebracht. Die einzelnen Blendenanordnungen 11 bzw. 12 besitzen ferner eine zum Rand der Blendenöffnungen hin abnehmende Dicke, so daß die Ränder der Perspektivbilder mehr oder weniger stark verwaschen sind.

Aus den kodierten Bildern können nach bekannten Dekodierverfahren (DE-B-27 19 386 und 27 46 035) durch Maßstabsänderung und Korrelation Schichten des dreidimensionalen Objektes gewonnen werden. Alle kodierten Bilder können dabei mit der gleichen Dekodiervorrichtung nacheinander ausgewertet werden. Eine grobe Objektorientierung ist dann mit den aus den stark überlagerten Perspektivbildern 7a rekonstruierten Schichtbildern möglich, während detaillierte und sichere Diagnosen mit den aus den weniger oder gar nicht überlagerten Perspektivbildern 7b bzw. 7c rekonstruierten Schichtbildern gestellt werden können. Vorausgesetzt ist natürlich, daß das gewünschte Objektdetail auch in dem vorhandenen Bildersatz aufgezeichnet wurde. Durch sog. Übersichtsaufnahmen, die häufig in der Radiologie gemacht werden, ist aber eine grobe Objektorientierung für die Aufnahme des Satzes der kodierten Bilder mit der Mehrfachstrahlenquelle möglich.

**Ansprüche**

1. Vorrichtung zur Erzeugung von Schichtbildern eines dreidimensionalen Objektes (5), mit in einer Strahlenquellenebene angeordneten Strahlenquellen (2, 3, 4) zur Durchstrahlung des Objektes mit aus unterschiedlichen Richtungen verlaufenden Strahlenbündeln (2a, 3a, 4a), und mit mehreren, unterhalb des Objektes und parallel zueinander angeordneten, ebenen Aufzeichnungsschichten (8, 9, 10) zur Aufnahme jeweils aller Perspektivbilder, dadurch gekennzeichnet, daß vor jeweils einer Aufzeichnungsschicht (8, 9, 10) eine ihr zugeordnete Blendenanordnung (11, 12) zur stufenweisen Ausblendung der Strahlenbündel angeordnet ist, wobei die Blendenöffnungen jeweils einer Blendenanordnung umso kleiner sind, je weiter die Blendenanordnung vom Objekt (5) entfernt ist.

2. Vorrichtung nach Anspruch 1, dadurch gekennzeichnet, daß jeweils eine Blendenanordnung (11, 12) gleich große Blendenöffnungen besitzt, die zentrisch zu den Strahlenbündeln liegen.

3. Vorrichtung nach Anspruch 1, dadurch gekennzeichnet, daß die Blendenanordnungen (11, 12) Blendenöffnungen besitzen, die sich in ihrer Größe, Form und Lage voneinander unterscheiden.

4. Vorrichtung nach Anspruch 2 oder 3, dadurch gekennzeichnet, daß die Blendenanordnungen (11, 12) als Blendenplatten ausgebildet sind.

5. Vorrichtung nach einem oder mehreren der Ansprüche 1 bis 4, dadurch gekennzeichnet, daß die Blendenanordnungen gegen den Rand der Blendenöffnungen in ihrer Dicke abnehmen.

6. Vorrichtung nach einem oder mehreren der Ansprüche 1 bis 5, dadurch gekennzeichnet, daß die Blendenanordnungen zusammen mit den Aufzeichnungsschichten in einer lichtdichten Kassette angeordnet sind.

7. Vorrichtung nach einem oder mehreren der Ansprüche 1 bis 6, dadurch gekennzeichnet, daß eine Filmwechselvorrichtung zur Positionierung der Kassette bzw. der Aufzeichnungsträger in ihrer jeweiligen Aufnahmeposition vorgesehen ist.

8. Vorrichtung nach einem oder mehreren der Ansprüche 1 bis 7, dadurch gekennzeichnet, daß die Aufzeichnungsschichten als Filme ausgebildet sind, die an beiden Seiten Verstärkerfolien mit jeweils unterschiedlicher Verstärkung tragen.

## Claims

1. A device for generating tomographic images of a three-dimensional object (5), comprising radiation sources (2, 3, 4) arranged in a radiation source plane for irradiating the object with beams of rays (2a, 3a, 4a) from different directions, and also comprising several planar recording layers (8, 9, 10) which are arranged beyond the object and parallel to one another each for recording all the perspective images, characterized in that in front of each recording layer (8, 9, 10) there is located an associated diaphragm arrangement (11, 12) for masking the beams of rays in a stepwise manner, the diaphragm apertures of each successive diaphragm arrangement being made smaller in dependence on the increase in the distance between the diaphragm arrangement and the object (5).

2. A device as claimed in Claim 1, characterized in that a diaphragm arrangement (11, 12) in each case has respectively diaphragm apertures of the same size which are each centrally located with respect to the corresponding beams of rays.

3. A device as claimed in Claim 1, characterized in that the diaphragm arrangements (11, 12) have diaphragm apertures that differ from one another in size, shape and position.

4. A device as claimed in Claim 2 or 3, characterized in that the diaphragm arrangements (11, 12) are in the form of diaphragm plates.

5. A device as claimed in any one of Claims 1 to 4, characterized in that the diaphragm arrangements decrease in thickness towards the edge of the diaphragm apertures.

6. A device as claimed in any one of Claims 1 to 5, characterized in that the diaphragm arrangements, together with the recording layers, are arranged in a light-tight cassette.

7. A device as claimed in any one of Claims 1 to 6, characterized in that a film-changing device is provided for positioning the cassette or the record carrier in their respective exposure positions.

8. A device as claimed in any one of Claims 1 to 7, characterized in that the recording layers are in the form of photographic films which are provided on both sides with an intensifier foil having an intensification which is different on each side.

## Revendications

1. Dispositif générateur d'images tomographiques d'un objet tridimensionnel (5) comportant des sources de rayons (2, 3, 4) disposées dans un plan de sources de rayons pour que l'objet soit traversé par des faisceaux de rayons (2a, 3a, 4a) s'étendant dans des directions différentes et comportant plusieurs couches d'enregistrement (8, 9, 10) planes parallèles les unes aux autres, disposées en dessous de l'objet et destinées à enregistrer chaque fois toutes les expositions en perspective, caractérisé en ce qu'un système de diaphragmes (11, 12) est installé devant chaque couche d'enregistrement (8, 9, 10) et y est associé pour diaphragmer cran par cran le faisceau de rayons, les ouvertures des systèmes de diaphragmes étant d'autant plus petites que le système de diaphragmes est davantage éloigné de l'objet (5).

2. Dispositif suivant la revendication 1, caractérisé en ce que chaque système de diaphragmes (11, 12) possède des ouvertures d'égales dimensions qui sont centrées par rapport aux faisceaux de rayons.

3. Dispositif suivant la revendication 1, caractérisé en ce que les systèmes de diaphragmes (11, 12) comportent des ouvertures qui se distinguent les unes des autres quant à leurs dimensions, leur forme et leur situation.

4. Dispositif suivant la revendication 2 ou 3, caractérisé en ce que les systèmes de diaphragmes (11, 12) ont la forme de plaques à diaphragmes.

5. Dispositif suivant l'une quelconque des revendications 1 à 4, caractérisé en ce que les systèmes de diaphragmes ont une épaisseur qui va en diminuant en direction du bord des ouvertures.

6. Dispositif suivant l'une quelconque des revendications 1 à 5, caractérisé en ce que les systèmes de diaphragmes sont disposés avec les couches d'enregistrement dans une cassette étanche à la lumière.

7. Dispositif suivant l'une quelconque des revendications 1 à 6, caractérisé en ce qu'un dispositif de changement de film est prévu pour positionner la cassette ou le support d'enregistrement dans chaque position d'exposition.

8. Dispositif suivant l'une quelconque des revendications 1 à 7, caractérisé en ce que les couches d'enregistrement ont la forme de films qui portent des deux côtés des écrans renforçateurs présentant des degrés de renforcement différents.

Fig.1